Europäisches Patentamt

European Patent Office

Office européen des brevets

(11) Veröffentlichungsnummer: **0 243 585 B1**

(12) **EUROPÄISCHE PATENTSCHRIFT**

(45) Veröffentlichungstag der Patentschrift: **02.01.92**

(21) Anmeldenummer: **87101241.5**

(22) Anmeldetag: **29.01.87**

(51) Int. Cl.⁵: **A61F 2/36**

(54) In einem Knochenhohlraum zu implantierender Prothesenschaft und Prothese mit dem Schaft.

(30) Priorität: **30.04.86 CH 1802/86**

(43) Veröffentlichungstag der Anmeldung:
**04.11.87 Patentblatt 87/45**

(45) Bekanntmachung des Hinweises auf die
Patenterteilung:
**02.01.92 Patentblatt 92/01**

(84) Benannte Vertragsstaaten:
**AT DE FR IT NL**

(56) Entgegenhaltungen:
**EP-A- 0 065 481**
**EP-A- 0 085 147**
**DE-A- 2 851 598**
**DE-A- 2 933 271**
**FR-A- 2 438 469**

(73) Patentinhaber: **Karpf, Kurt**
**Römerstrasse 175**
**CH-4718 Holderbank(CH)**

(72) Erfinder: **Karpf, Kurt**
**Römerstrasse 175**
**CH-4718 Holderbank(CH)**

(74) Vertreter: **Keller, René, Dr. et al**
**Patentanwälte Hartmut Keller, Dr. René Kel-**
**ler Postfach 12**
**CH-3000 Bern 7(CH)**

Rank Xerox (UK) Business Services

## Beschreibung

Die Erfindung bezieht sich auf einen in einem Knochenhohlraum, insbesondere im Markraum eines Röhrenknochens, zu implantierenden Prothesenschaft und auf eine Prothese, insbesondere Gelenkprothese, mit diesem Schaft.

Prothesenschäfte werden bei der Implantation in dem passend vorgeraspelten Knochenhohlraum fixiert, z.B. einzementiert oder für zementfreie Implantation zum freien Schaftende hin verjüngt ausgeführt und in den angepassten Hohlraum geschlagen, so dass sie durch Presssitz mit dem Knochen verbunden sind.

Es ist ein Prothesenschaft bekannt (DE-A-2 933 271), der hohl und mit Oeffnungen versehen ist, wobei zwischen benachbarten Oeffnungen Stege gebildet sind. Durch die Oeffnungen des implantierten Schaftes kann das Knochengewebe in den Schafthohlraum hineinwachsen, wodurch die Verbindung zwischen Schaft und Knochen verbessert wird. Auch kann der Schafthohlraum vor dem Implantieren des Prothesenschaftes mit künstlichem Knochenmaterial mindestens teilweise gefüllt werden.

Bei Belastung eines Knochens mit implantiertem Prothesenschaft biegt sich der Knochen zusammen mit dem Schaft. Dabei wird das Ganze an der konvexen Seite der Biegung auf Dehnung beansprucht und dadurch (im Mikrometerbereich) länger, an der konkaven Seite der Biegung auf Druck beansprucht und dadurch entsprechend kürzer. Bei dieser gemeinsamen Verformung von Knochen und Schaft entstehen an der Grenzfläche, zwischen Knochen und Schaft Schubkräfte, zusätzlich zu der Schubkraft, die durch die Uebertragung der Belastung vom Schaft auf den Knochen auftritt. Wenn die resultierende Schubkraft die Schubfestigkeit der Verbindung überschreitet, kommt es zu Verschiebungen an den Grenzflächen, der Schaft sitzt dann nur noch locker im Knochen, und eine Nachoperation wird erforderlich.

Die mit der Biegung zusammenhängende Schubkraft ist umso grösser, je länger die Strecke ist, an welcher der Schaft fest mit dem Knochen verbunden ist. Um diese Schubkraft klein zu halten, damit sie auch bei Belastungsspitzen zuverlässig unterhalb der Schubfestigkeit bleibt, beschränkt man die Länge der Verbindungsfläche auf einen Teil der Schaftlänge und führt den restlichen Teil des Schaftes mit glatter Oberfläche aus, so dass er gleitend verschiebbar im Knochen sitzt. Beispielsweise wird bei einem Hüftgelenk nur der proximale (der Körpermitte zugewandte) Teil der Schaftlänge mit dem Knochen fest verbunden, wobei der restliche (distale) Teil der Länge des Schaftes gleitend verschiebbar im Knochen bleibt. Dies, obwohl der Schaft grundsätzlich an einer möglichst grossen Fläche mit dem Knochen verbunden werden sollte, um mit einer möglichst kleinen, weit unter der Schubfestigkeit liegenden Schubbelastung pro Flächeneinheit der Grenzfläche auszukommen.

Die Biegsamkeit des Knochens mit dem implantierten Schaft ist in dem Längenbereich, in dem der Schaft im Knochen sitzt, wesentlich kleiner als im restlichen Längenbereich des Knochens. Bei Belastung kann der Knochen praktisch nur noch in dem restlichen Bereich elastisch nachgeben. Die Nachgiebigkeit des Knochens ist durch den implantierten Schaft herabgesetzt. Die geringere Nachgiebigkeit kann bei Belastungsspitzen zu einer Ueberlastung der Knochen-Schaft-Verbindung und zu Ueberlastungen und vorzeitiger Abnutzung des implantierten Gelenks führen.

Auch die plötzliche Aenderung der Biegsamkeit oder Biegungssteifigkeit an der Grenze zwischen dem den Schaft enthaltenden und dem restlichen Teil des Knochens ist unerwünscht und kann bei Belastungsspitzen sogar zu Knochenbrüchen führen.

Hier will die Erfindung Abhilfe schaffen. Die Erfindung, wie sie im Patentanspruch 1 gekennzeichnet ist, löst die Aufgabe, einen Prothesenschaft der genannten Art zu schaffen, mit dem ein Lockern des Schaftes durch Biegungsbeanspruchung und eine Herabsetzung der Biegsamkeit des Knochens vermieden werden kann, auch wenn der Schaft an einem grösseren Längsabschnitt oder an seiner ganzen Länge fest mit dem Knochen verbunden ist.

Die durch die Erfindung erzielten Vorteile sind im wesentlichen darin zu sehen, dass die Belastung an einer wesentlich längeren Grenzfläche vom Schaft auf den Knochen übertragen werden kann. Dabei ist die Schubkraft pro Grenzflächeneinheit kleiner. Bei einem Presssitz genügt für die erforderliche Haftreibung ein kleinerer radialer Druck. Das hat deshalb besondere Bedeutung, weil bei natürlicher Belastung (d.h. mit dem natürlichen Gelenk) weder eine solche Schubkraft an der Knocheninnenwand noch ein radialer Druck vorkommt, so dass die Knochenstruktur von Natur aus nicht für diese Beanspruchungen bestimmt ist. Weiterhin wird die Biegsamkeit des Knochens durch den erfindungsgemässen Schaft wesentlich weniger herabgesetzt, so dass der Knochen bei Belastung im wesentlichen an seiner ganzen Länge elastisch nachgeben kann. Im Zusammenhang damit ist auch die Aenderung der Biegsamkeit an der Grenze zwischen dem den Schaft enthaltenden und dem restlichen Teil des Knochens vernachlässigbar. Ausführungsarten der Erfindung sind in den abhängigen Patentansprüchen angegeben, die durch sie erzielten Vorteile gehen aus der folgenden Beschreibung hervor.

Die Erfindung wird im folgenden anhand von lediglich Ausführungsarten darstellender Zeichnungen näher erläutert.

Es zeigen

Fig. 1 einen Längsschnitt durch einen oberen (proximalen) Teil eines Oberschenkelknochens, in den ein teils ausgebrochen dargestellter Schaft einer Hüftgelenkprothese implantiert ist,

Fig. 2 eine perspektivische Ansicht des Schaftes von Fig. 1,

Fig. 3 bis 5 verschiedene Querschnittsformen des Schaftes von Fig. 1 und 2.

Fig. 6 bis 8 Varianten der Form der Oeffnungen des Schaftes von Fig. 1 und 2,

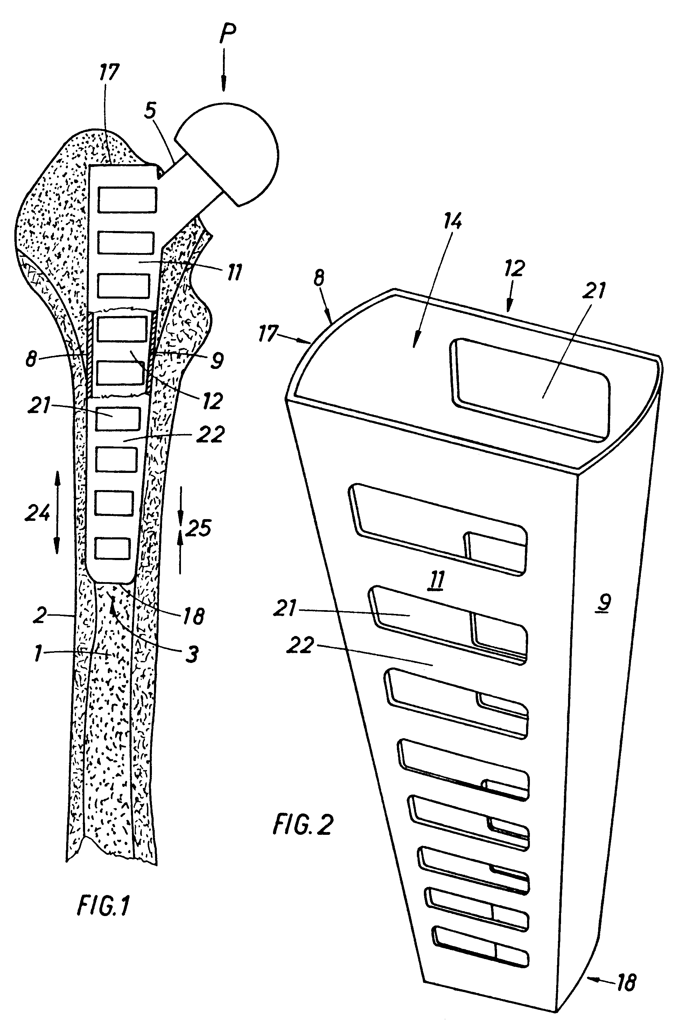

In Fig. 1 ist eines der Gelenkglieder einer Hüftgelenkprothese dargestellt, das in seinem grundsätzlichen Aufbau aus einem in einen proximalen Teil des Markraums 1 eines nur teilweise dargestellten Oberschenkelknochens 2 eingesetzten Schaft 3 und einer Gelenkkugel 4 besteht, die durch Pressitz mit einem Ende eines Ansatzes 5 des Schaftes 3 auswechselbar verbunden ist.

Erfindungsgemäss ist der Schaft 3 hohl, wie z.B. aus Fig. 1 und 2 hervorgeht, wo die Schaftwand an der lateralen (der Mittellinie des Rumpfes des Patienten abgewandten) Seite mit 8, an der medianen (dieser Mittellinie zugewandten) Seite mit 9, an der Vorderseite mit 11, an der Rückseite mit 12 und der Hohlraum des Schaftes 3 mit 14 (Fig. 2) bezeichnet sind. Der Querschnitt des Schaftes 3 ist länglich, in Fig. 1 und 2 im wesentlichen rechteckig, wobei die laterale und die mediale Schaftseite 8 und 9 die Schmalseiten, die vordere und die hintere Schaftseite 11 und 12 die Breitseiten bilden. Nur die Schmalseiten 8 und 9 des Schaftes liegen am Knochen 2 an, und zwar an ihrer ganzen Länge, sie konvergieren vom proximalen (rumpfwärts gelegenen) Ende 17 zum distalen (weiter vom Rumpf entfernten) Ende 18 des Schaftes 3 und sind leicht nach aussen gewölbt. Der Markhohlraum 1 des Knochens 2 ist so bearbeitet (geraspelt), dass die an der lateralen und an der medianen Seite 8 und 9 des Schaftes 3 anliegenden Knochenflächen entsprechend gewölbt sind und entsprechend konvergieren. Der Schaft 3 ist zwischen diesen Flächen in den Markraum 1 getrieben, so dass er durch Pressitz fest mit dem Knochen 2 verbunden ist. An der vorderen und der hinteren Seite 11 und 12 ist der Schaft 3 mit in Fig. 1 und 2 rechteckigen Oeffnungen 21 versehen, zwischen denen Stege 22 gebildet sind, welche den lateralen Teil 8 der Schaftwand mit dem medialen 9 verbinden und im Abstand voneinander halten.

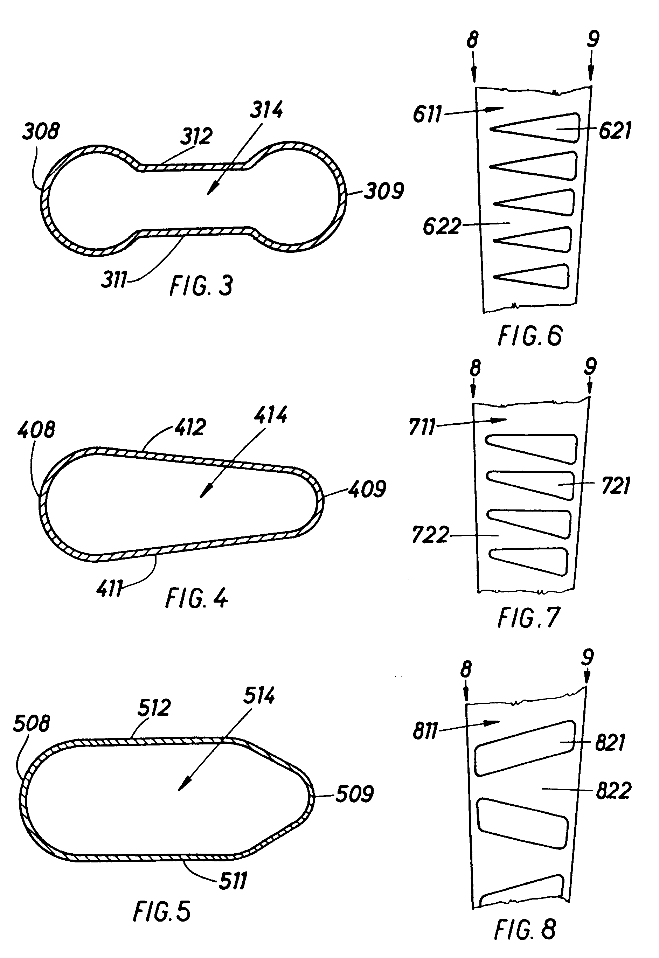

Fig. 3, 4 und 5 zeigen drei Varianten des Querschnitts ses Schaftes, wobei der in Fig. 2 mit 14 bezeichnete Schafthohlraum bei der Variante nach Fig. 3 mit 314, bei der Variante nach Fig. 4 mit 414 und bei der Variante nach Fig. 5 mit 514 bezeichnet ist, und die Bezugszahlen der Schaftseiten auf dieselbe Weise aus der Nummer der Figur und den Bezugszahlen 8, 9, 12 und 13 von Fig. 1 und 2 gebildet sind. Die Figuren 6, 7 und 8 zeigen Varianten der Form der in Fig. 1 und 2 dargestellten Oeffnungen 21 und Stege 22 der Schaftvorder- und -rückseite 11 und 12, wobei auf dieselbe Weise die Schaftvorderwand mit 611, 711 bzw. 811, die Varianten der Oeffnungen entsprechend mit 621, 721 bzw. 821 und die Stege mit 622, 722 und 822 bezeichnet sind. Die Oeffnungen und die Stege an der Rückseite sind gleich denen an der Vorderseite.

Eine Belastung P des Gelenks übt über den als Hebel wirkenden Ansatz 5 ein Biegemoment auf den Knochen 2 und den mit ihm verbundenen Schaft 3 aus. Dadurch werden beide an der konvexen, lateralen Seite der Krümmung auf Dehnung (Doppelpfeil 24) und an der medianen, konkaven Seite auf Druck (Pfeile 25) beansprucht, wobei sowohl die Dehnungsverformung (24) als auch die Druckverformung (25) des Knochens 2 grösser sind als die des Schaftes 3. Als Folge davon tritt in der Fläche, an der Knochen und Schaft fest miteinander verbunden sind, eine Schubkraft auf, Knochen 2 und Schaft 3 sind bestrebt, sich aneinander zu verschieben. Eine solche Verschiebung, die den Schaft 3 im Knochen 2 lockern würde, muss durch eine genügend feste Verbindung, im Falle eines Pressitzes des Schaftes 3 im Knochen 2 durch eine genügende Haftreibung verhindert werden. Die Schubkraft ist umso grösser, je kleiner die Dehnbarkeit des Schaftes 3 und je länger (in Längsrichtung von Schaft 3 und Knochen 2) die Fläche ist, an der der Schaft 3 und der Knochen 2 miteinander verbunden sind. Weil der erfindungsgemässe Schaft 3 hohl ist, ist er dehnbarer als die bekannten, massiven Schäfte, und seine Dehnbarkeit ist durch Bemessung der Wandstärke beeinflussbar. Dadurch ist es möglich, den Schaft 3 so auszuführen, dass er für die Uebertragung der Belastung P vom Schaft 3 an den Knochen 2 genügend stabil, jedoch an seiner lateralen und seiner medianen Seite möglichst dehnbar ist, um die infolge der Belastung auftretenden Schubkräfte an der Grenzfläche von Schaft und Knochen möglichst klein zu halten. Dadurch kann das Risiko eines Lockerns des Schaftes 3 wesentlich herabgesetzt werden, und im Falle eines Pressitzes des Schaftes 3 im Knochen 2 genügt ein kleinerer radialer Druck auf den Knochen. Wegen der kleineren Schubkraft kann der Schaft 3 ohne weiteres an seiner ganzen Länge mit dem Knochen 2 verbunden werden. Wegen der grösseren Dehnbarkeit des Schaftes 3 unterscheidet sich die Biegsamkeit des Knochens 2 an dem Längsabschnitt, an dem Knochen 2 und Schaft 3 fest miteinander verbunden sind, nicht wesentlich von der Biegsamkeit des Knochens 2 im restlichen Längsabschnitt.

Auch die Oeffnungen 21, 621, 721, 821 erhöhen die Dehnbarkeit der Schaftwand 8 an der lateralen Seite und 9 an der medianen Seite, weil sie eine Scherungsverformung der vorderen und hinteren Schaftwand, 11, 311, 411, 511 und 12, 312, 412, 512 erleichtern, wobei die Stege 22, 622, 722, 822 dazu dienen, die laterale und die mediale Schaftwand in erforderlichen Abstand voneinander zu halten.

Der Schaft 3 kann aus Metall, einer Metallegierung oder aus Kunststoff, insbesondere verstärktem Kunststoff, bestehen und mit einem gewebefreundlichen Material, z.B. Titan oder Hydroxylapatit, überzogen sein.

Am proximalen Schaftende können zusätzlich Verankerungselemente in den Schafthohlraum eingebracht und dort durch die Oeffnungen des Schaftes hindurch im Knochen verankert werden. Auch können an diesem Schaftende Mark und Knochenspäne, insbesondere das zur Vorbereitung der Implanation aus dem Knochenhohlraum entfernte Mark und die beim Raspeln der Knocheninnenseite angefallenen Knochenspäne, in den Schafthohlraum eingebracht werden, um durch die Oeffnungen des Schaftes hindurch in Verbindung mit dem Knochen zu kommen. Dadurch bleibt auch im Bereiche des implantierten Schaftes das natürliche Zusammenwirken von Knochen und Knochenmark erhalten. Unabhängig davon ist eine Neubildung von Knochen und/oder Knochenmark im Bereiche der Oeffnungen und in den Schafthohlraum hinein ermöglicht.

## Patentansprüche

1. In einem Knochenhohlraum (17) zu implantierender Prothesenschaft (3), der mindestens am grössten Teil seiner Länge hohl ist und mit Oeffnungen (21) versehen ist, wobei zwischen benachbarten Oeffnungen Stege (22) gebildet sind, dadurch gekennzeichnet, dass der Schaft (3) zwei schmalere Seiten (8, 9), deren eine (8) die laterale und deren andere (9) die mediane Seite bildet, und zwei breitere Seiten (11, 12) hat, wobei nur die schmaleren Seiten (8, 9) dazu bestimmt sind, an ihrer ganzen Länge fest mit dem Knochen (2) in Anlage zu kommen und die breiteren Seiten (11, 12) mit den Oeffnungen (21) versehen sind, und dass die Stege (22) die schmaleren Seiten (8, 9) des Schaftes (3) oder an diese angrenzende Teile der breiteren Seiten (11, 12) im Abstand voneinander halten.

2. Schaft nach Anspruch 1, dadurch gekennzeichnet, dass er (3) aus Metall, Metallegierung oder Kunststoff, insbesondere verstärktem Kunststoff, besteht.

3. Schaft nach Anspruch 1 oder 2, dadurch gekennzeichnet, dass er (3) mit einem gewebefreundlichen Material überzogen ist.

4. Prothese mit dem Schaft nach einem der Ansprüche 1 bis. 3.

## Claims

1. A prosthesis stem (3) which is adapted to be implanted in a bone cavity (17) and which over at least the major part of its length is hollow and provided with apertures (21), webs (22) being formed between adjacent apertures, characterised in that the stem (3) has two narrower sides (8, 9), of which one (8) is the lateral side while the other (9) is the median side, and two broader sides (11, 12), only the narrower sides (8, 9) being intended to have their entire length rigidly in contact with the bone (2), the broader sides (11, 12) being provided with the apertures (21), and in that the webs (22) maintain the narrower sides (8, 9) of the stem (3) or parts of the broader sides (11, 12) adjacent thereto at a distance from one another.

2. A stem according to claim 1, characterised in that the stem (3) consists of metal, metal alloy or synthetic plastics material, in particular reinforced synthetic plastics material.

3. A stem according to claim 1 or 2, characterised in that the stem (3) is coated with a tissue-compatible material.

4. A prosthesis comprising the stem according to one of claims 1 to 3.

## Revendications

1. Tige de prothèse (3) destinée à être implantée dans le canal (17) d'un os, qui est creuse sur au moins la plus grande partie de sa longueur et qui est munie d'ouvertures (21), des entretoises (22) étant formées entre les ouvertures voisines, caractérisée en ce que la tige (3) présente deux côtés plus étroits (8, 9) dont l'un (8) forme le côté latéral et dont l'autre (9) forme le côté médian, et deux côtés plus larges (11, 12), seuls les côtés plus étroits (8, 9) étant destinés à venir fermement en appui avec l'os (2) sur toute leur longueur et les côtés plus larges (11,12) étant munis des ouvertures (21), et en ce que les entretoises (22) maintiennent écartés les côtés plus étroits (8,9) de la tige (3) ou les parties voisines de ceux-ci des côtés plus larges (11,12).

2. Tige selon la revendication 1, caractérisée en ce qu'elle (3) consiste en métal, en alliage métallique ou en matière plastique, en particulier en matière plastique renforcée.

3. Tige selon la revendication 1 ou 2, caractérisée en ce qu'elle (3) est recouverte d'une matière compatible avec les tissus.

4. Prothèse avec la tige selon l'une des revendications 1 à 3.

FIG.1

FIG.2

FIG. 3

FIG. 4

FIG.5

FIG.6

FIG.7

FIG. 8